# EUROPÄISCHE PATENTSCHRIFT

(11) **EP 3 873 322 B1**
(45) Veröffentlichungstag und Bekanntmachung des Hinweises auf die Patenterteilung: **23.11.2022**
(21) Anmeldenummer: 19798211.9
(22) Anmeldetag: 30.10.2019
(51) Int. Cl.: A61B 3/024, A61B 3/06

(54) **ORTSBEZOGENE QUANTIFIZIERUNG VON HALO- UND STREULICHT-BEEINTRÄCHTIGUNG**
LOCATION-BASED QUANTIFICATION OF IMPAIRMENT DUE TO HALO AND SCATTERED LIGHT
QUANTIFICATION, BASÉE SUR LA LOCALISATION, DE L'IMPACT D'UNE LUMIÈRE HALOGÈNE OU DIFFUSÉE

(30) Priorität: 31.10.2018 DE 102018127299; 09.08.2019 DE 102019121602
(43) Veröffentlichungstag der Anmeldung: 08.09.2021
(73) Patentinhaber: Hochschule Aalen, 73430 Aalen (DE)
(72) Erfinder: SCHIEFER, Ulrich, 72127 Kusterdingen (DE); UNGEWISS, Judith, 73430 Aalen (DE); WOERNER, Michael, 70199 Stuttgart (DE)
(74) Vertreter: DREISS Patentanwälte PartG mbB
(86) Internationale Anmeldenummer: PCT/EP2019/079628
(87) Internationale Veröffentlichungsnummer: WO 2020/089284

(56) Entgegenhaltungen:
- WO-A1-2017/165373
- US-A1- 2012 162 606
- US-A1- 2015 342 459

## Beschreibung

Die vorliegende Erfindung betrifft ein Verfahren zur Akquisition von Daten, die eine Blendungsempfindlichkeit des Sehsinns einer Testperson kennzeichnen nach dem Oberbegriff des Anspruchs 1 sowie eine Vorrichtung nach dem Oberbegriff des unabhängigen Vorrichtungsanspruchs. Ein solches Verfahren und eine solche Vorrichtung sind jeweils aus der US 2014/0036232 bekannt.

Bei dem bekannten Verfahren wird eine Testvorrichtung bereitgestellt, die eine Präsentationsfläche aufweist, auf der Sehzeichen in variablen Positionen und wenigstens eine ortsfeste Blickfixierungsmarkierung darstellbar sind. Eine Blendlichtquelle ist seitlich versetzt zu einer Blickrichtung der Testperson so angeordnet, dass mit ihr ein Lichtbündel erzeugbar ist, das die Testperson blendet, wenn die Blickrichtung zwischen einem vorbestimmten Ort der Testperson und der Blickfixierungsmarkierung verläuft.

Aus der US 2015/342 459 A1 ist eine Testvorrichtung und - Verfahren zur Akquisition von Daten, die sich auf eine Blendungsempfindlichkeit einer Testperson beziehen, bekannt, wobei die Testvorrichtung eine Präsentationsfläche zum Darstellen einer Blickfixierungsmarkierung und mehrerer Sehzeichen, eine Blendlichtquelle und eine Eingabevorrichtung zur Erfassung von Reaktionen der Testperson aufweist. Ein ähnlicher Gegenstand ist auch aus der US 2012/162 606 A1 bekannt. Die Druckschrift WO2017/165373 A1 offenbart eine Augentestvorrichtung mit einem Display zur Darstellung von Sehzeichen und eine Augenverfolgungsvorrichtung, wobei Sehzeichen erst dann angezeigt werden, wenn die Augen der Testperson auf ein kontinuierlich angezeigtes Element gerichtet sind.

Als Foveales Sehen bezeichnet man Sehen in einer bestimmten Blickrichtung mit unbewegtem Blick geradeaus innerhalb eines sehr kleinen Raumwinkels von 1° bis 2°. Das Foveale Sehen erlaubt die Fixierung eines Sehobjekts sowie eine Erkennung von Konturen, Entfernungen und kleinsten Helligkeits- und Farbunterschieden in kurzer Zeit, was auch als Sehrohreffekt bekannt ist.

Davon unterscheidet man das Periphere Sehen. Unter der "Peripherie" wird in diesem Zusammenhang häufig nur der Gesichtsfeldbereich außerhalb einer Exzentrizität von 30° bezeichnet. In der vorliegenden Anmeldung wird unter dem Peripheren Sehen das Sehen außerhalb einer Blickachse speziell in dem "intermediären" Bereich zwischen ca. 2° und ca. 30° zur Blickachse verstanden. Das Periphere Sehen liefert Informationen über den Raum und eröffnet die Möglichkeit, den Raum wahrzunehmen.

Die vorliegende Erfindung betrifft die Aufgabe, ein Verfahren und eine Vorrichtung zur Akquisition von Daten anzugeben, die eine Blendungsempfindlichkeit des Sehsinns einer Testperson beim Peripheren Sehen kennzeichnen.

Diese Aufgabe wird in ihren Verfahrensaspekten durch die kennzeichnenden Merkmale des Anspruchs 1 und in ihren Vorrichtungsaspekten durch die Merkmale des unabhängigen Vorrichtungsanspruchs gelöst.

Das Verfahren zeichnet sich danach durch ein Bereitstellen einer Eingabevorrichtung, mit der Reaktionen der Testperson erfassbar und aufzeichnungsbar sind und einer Blickrichtungserkennungsvorrichtung aus, mit der eine Blickrichtung der Testperson detektierbar ist. Bei der Durchführung des Verfahrens wird eine Testperson so platziert, dass die Blickfixierungsmarkierung in einem zentralen Bereich des Sichtfeldes der Testperson liegt. Die Testperson wird aufgefordert, ihren Blick auf die Blickfixierungsmarkierung zu richten und nicht von der Blickfixierungsmarkierung zu lösen. Im Folgenden wird die Blickrichtung der Testperson fortwährend mit der Blickrichtungserkennungsvorrichtung überwacht. Darüber hinaus wird die Testperson dazu aufgefordert, eine bestimmte Reaktion zu zeigen, wenn sie ein Sehzeichen auf der Präsentationsfläche wahrnimmt.

Weiter wird die Blendlichtquelle eingeschaltet. Bei eingeschalteter Blendlichtquelle erfolgt ein Darstellen von Sehzeichen in verschiedenen Positionen einer vorbestimmten Bahnkurve auf der Präsentationsfläche. In einer ersten Alternative werden die Sehzeichen kontinuierlich entlang der Bahnkurve bewegt dargestellt. In einer zweiten Alternative werden die Sehzeichen an diskreten Punkten (also z.B. einem sog. Untersuchungsraster oder einem sog. Profilschnitt) jeweils statisch für eine vorbestimmte Präsentationsdauer dargestellt, also gewissermaßen in diskreten Schritten von einem statischen Darstellungsort zum nächsten statischen Darstellungsort bewegt. Weiter erfolgt eine Überwachung der Testperson auf das Zeigen der bestimmten Reaktion. Wenn mit der Blickrichtungserkennungsvorrichtung erkannt wird, dass sich der Blick der Testperson zwischen der Darstellung des Sehzeichens und der davon ausgelösten Reaktion der Testperson von der Blickfixierungsmarkierung gelöst hat, wird die Darstellung des Sehzeichens abgebrochen.

Dagegen erfolgt ein Erfassen und Speichern von Daten, welche die Position des Sehzeichens in dem Moment kennzeichnen, in dem die Testperson die Reaktion zeigt, dann und nur dann, wenn sich der Blick der Testperson zwischen der Darstellung des Sehzeichens und der davon ausgelösten Reaktion der Testperson nicht von der Blickfixierungsmarkierung gelöst hat.

Diese Merkmale erlauben eine Vorgabe standardisierter und reproduzierbarer Randbedingungen für die Akquisition der oben genannten Daten. Die unter diesen Bedingungen akquirierten Daten erlauben eine Beurteilung ortsbezogener visueller Beeinträchtigungen des Sehsinns der Testperson durch Halo oder Streulicht. Durch mehrfache Wiederholung der Darstellung von Sehzeichen längs gleicher Bahnkurven oder an gleichen Punkten eines Rasters und statistische Bewertung der jeweiligen Reaktionen ist eine Erfassung der intra-individuellen Streuung und der interindividuellen Streuung möglich. Ein wesentlicher Vorteil der Erfindung besteht in der Möglichkeit der Herstellung eines Ortsbezugs bei der Akquisition der Daten durch Referenzieren auf eine Zahl und Anordnung von Blendlichtquellen.

Die Erfindung ermöglicht insbesondere auch eine Erfassung der Wirkung von optischen Korrekturen (z.B. durch Kunstlinsen, wie Presbyopie-korrigierende Kontakt- oder Intraokularlinsen (also ins Auge des Menschen eingesetzte künstliche Linsen, die entweder zusätzlich zur menschlichen Linse platziert werden oder die menschliche Linse ersetzen) oder Hornhaut-chirurgische Maßnahmen) und von Korrektionsmitteln (äußerlich wirksame Maßnahmen:
Gleitsichtgläser, Nachtsichtgläser, Brillen, Kontaktlinsen etc.) über das gesamte Sehfeld).

Die Erfindung erlaubt eine Akquisition von Daten, die eine örtlich aufgelöste Erfassung der Niedrigkontrast-Sehschärfeverteilung bzw. Kontrastempfindlichkeitsverteilung kennzeichnen.

Eine bevorzugte Ausgestaltung zeichnet sich dadurch aus, dass die Blendlichtquelle bezüglich einer auf die Blickfixierungsmarkierung gerichteten Blickachse der Testperson seitlich versetzt in einem Bereich des Peripheren Sehens positioniert wird.

Bevorzugt ist auch, dass die Blendlichtquelle so positioniert wird, dass sie eine Exzentrische/Para-zentrale Blendung erzeugt.

Weiter ist bevorzugt, dass das Sehzeichen einen 8-Positionen Landolt-Ring und/oder ein davon verschiedenes grafisches Symbol und/oder wenigstens eine Zahl und/oder wenigstens einen Buchstaben aufweist.

Eine weitere bevorzugte Ausgestaltung zeichnet sich dadurch aus, dass das Sehzeichen mit vorgegebener Geschwindigkeit kontinuierlich entlang einer Bahnkurve bewegt wird, deren Ursprung von der Testperson aus gesehen von der Blendlichtquelle verdeckt wird. Wie weiter oben erwähnt, ist das Verfahren auch mit örtlich diskreten, statischen Darstellungen durchführbar. Dann erfolgt eine Abfolge räumlich diskreter Darstellungen mit vorgegebener Schrittgeschwindigkeit.

Weiter ist bevorzugt, dass aus den längs jeweils einer Bahnkurve erfassten Daten durch statistische Bewertung wie Mittelwertbildung oder Medianbildung von Schwellenwertabständen für diese Bahnkurve ein Punkt bestimmt wird, dessen Abstand vom Schnittpunkt für diese Bahnkurven eine Entfernung kennzeichnet, bis zu der die Blendlichtquelle für die betreffende Testperson eine Erkennung des Sehzeichens verhindert hat. Der Schwellenwertabstand ist dabei der Abstand des Sehzeichens von dem Bahnkurvenursprung bei einem einmaligen Durchlaufen der Bahnkurve. Ggf. werden mit einem mehrmaligen Durchlaufen der Bahnkurve mehrere Schwellenwertabstände bestimmt, die dann statistisch ausgewertet werden (z.B. durch Mittel- und/oder Medianbildung).

Eine weitere bevorzugte Ausgestaltung zeichnet sich dadurch aus, dass bei der Bestimmung der Daten eine Reaktionszeit der Testperson berücksichtigt wird.

Bevorzugt ist auch, dass die Berücksichtigung dadurch erfolgt, dass der Teil der Bahnkurve, den das Sehzeichen in der Reaktionszeit zurückgelegt als Produkt aus Reaktionszeit und Sehzeichengeschwindigkeit bestimmt wird und dass der zunächst ohne Berücksichtigung der Reaktionszeit bestimmte Punkt der Bahnkurve um diesen Teil der Bahnkurve in Richtung zu der initialen Position verlegt wird.

Weiter ist bevorzugt, dass die Schrittfolge des Anspruchs 1 beendet wird, wenn sich der Blick der Testperson von der Blickfixierungsmarkierung löst und alle ggf. bereits für dieses Sehzeichen festgestellten Wahrnehmungen/Reaktionen verworfen werden.

Eine weitere bevorzugte Ausgestaltung zeichnet sich dadurch aus, dass das Darstellen eines Sehzeichens für eine vorbestimmte Präsentationsdauer erfolgt und dass dann, wenn innerhalb der Präsentationdauer keine Reaktion der Testperson erfolgt, für die betreffende Präsentationsdauer auch keine Daten akquiriert werden.

Bevorzugt ist auch, dass die Position der Blendlichtquelle bei einer Durchführung des Verfahrens nach einer bei einer ersten Blendlichtquellenposition erfolgten Akquisition von Daten verändert wird und das Verfahren für verschiedene Positionen der Blendlichtquelle durchgeführt wird. Die Vorrichtung zeichnet sich entsprechend dadurch aus, dass die Position der Blendlichtquelle verändert werden kann.

Weiter ist bevorzugt, dass die spektrophoto-radiometrischen Eigenschaften, also die Eigenschaften der von der Blendlichtquelle ausgehenden Strahlung variiert werden, und dass eine Akquisition von Daten für verschiedene spektrophoto-radiometrische Eigenschaften der Blendlichtquelle erfolgt.

Weitere Vorteile ergeben sich aus der nachfolgenden Beschreibung, den Zeichnungen und den Unteransprüchen. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen. Ausführungsbeispiele der Erfindung sind in den Zeichnungen dargestellt und werden in der nachfolgenden Beschreibung näher erläutert.

Dabei zeigen, jeweils in schematischer Form:
Figur 1 ein Ausführungsbeispiel einer erfindungsgemäßen Vorrichtung;
Figur 2 eine grafische Veranschaulichung von mit der Erfindung akquirierbarer Daten;
Figur 3 eine grafische Darstellung von zu einem Diagramm verarbeiteten Daten; und
Figur 4 ein Flussdiagramm als Ausführungsbeispiel eines erfindungsgemäßen Verfahrens.

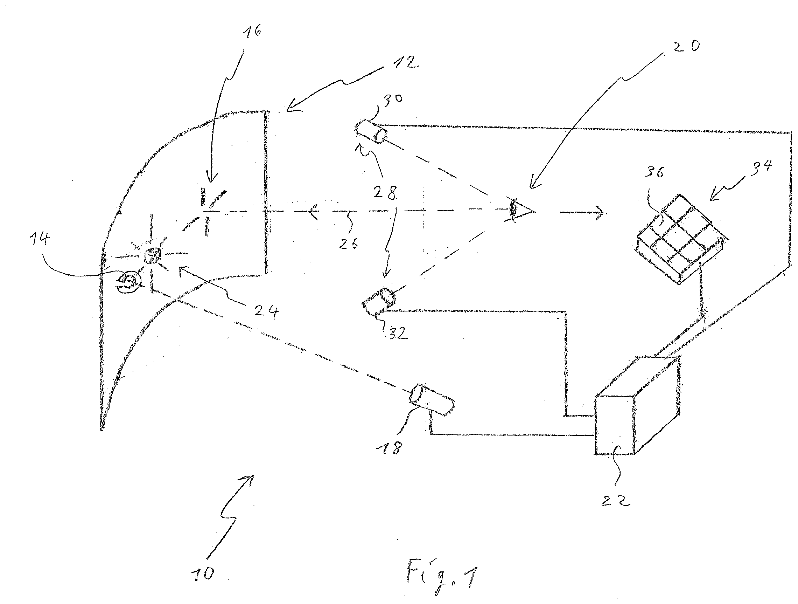

Im Einzelnen zeigt die Figur 1 eine Testvorrichtung 10 mit einer Präsentationsfläche 12, auf der visuelle Stimuli in Form von Sehzeichen 14 in variablen Positionen zusammen mit einer ortsfesten Blickfixierungsmarkierung 16 darstellbar sind.

Bei den Sehzeichen 14 kann es sich zum Beispiel um die bekannten Landolt-Ringe, zum Beispiel um 8-Positionen Landolt-Ringe handeln, ohne dass die Erfindung auf die Verwendung dieser speziellen Sehzeichen beschränkt ist. Als Alternative können zum Beispiel grafische Symbole und/oder Zahlen und/oder Buchstaben sowie andere Sehzeichen verwendet werden.

In einer bevorzugten Ausgestaltung sind die Sehzeichen 14 in Bezug auf wenigstens eine der Eigenschaften Sehwinkel (d.h. Größe), Kontrast, Präsentationsdauer, Orientierung (z.B. Lage einer Lücke in einem Landolt-Ring) und Art des Sehzeichens 14 (Buchstaben, Landolt-Ringe etc.) variabel.

Die Präsentationsfläche 12 kann zum Beispiel eine Projektionsfläche sein, auf der die Sehzeichen 14 in variablen Positionen und Helligkeiten zusammen mit wenigstens einer ortsfesten Blickfixierungsmarkierung 16 darstellbar sind. In einer bevorzugten Ausgestaltung dient ein Projektor 18 zur Projektion von Sehzeichen 14 auf die Präsentationsfläche 12. Die Präsentationsfläche 12 kann aber auch als ein Bildschirm eines elektrischen Bildschirmgerätes verwirklicht sein. Die Präsentationsfläche 12 ist bevorzugt um den Ort 20, an dem sich die Testperson während der Akquisition der Daten befindet, herum gewölbt. Der Projektor 18, bzw. das elektrische Bildschirmgerät, wird von einem Steuergerät 22 der Testvorrichtung 10 gesteuert.

Die Blickfixierungsmarkierung 16 kann ein stofflicher Bestandteil der Präsentationsfläche 12 sein oder von einem Projektor, der mit dem die Sehzeichen 14 projizierenden Projektor 18 identisch sein kann, auf die Präsentationsfläche 12 projiziert werden. Bei Verwendung eines Bildschirmgerätes wird die Blickfixierungsmarkierung 16 auf dem Bildschirm erzeugt.

Zwischen der Präsentationsfläche 12 und dem genannten Ort 20 der Testperson ist eine Blendlichtquelle 24 angeordnet. Die Blendlichtquelle 24 ist zum Beispiel eine näherungsweise punktförmige Leuchtdiode oder, was bevorzugt ist, eine Anordnung von mehreren Leuchtdioden, bevorzugt eine Anordnung von RGB-Leuchtdioden, mit denen bekanntlich Licht mit variabler Lichtfarbe erzeugbar ist. Durch eine z.B. matrixartige Anordnung von individuell steuerbaren Leuchtdioden ist der Sehwinkel (d.h. die Größe) der Blendlichtquelle 24 variierbar. Bevorzugt sind weitere optische Eigenschaften der Blendlichtquelle 24, wie deren Leuchtdichte und deren spektrale Eigenschaften, variierbar. Eine solche Variation kann alternativ oder ergänzend auch durch vorgeschaltete Filter- und Diffusorelemente/- folien erreicht werden.

Die Blendlichtquelle 24 ist bevorzugt unmittelbar vor der Präsentationsfläche 12 angeordnet, so dass ihr Abstand zum Ort 20 der Testperson im Wesentlichen gleich dem Abstand des Ortes 20 der Testperson von der Präsentationsfläche 12 ist. Die Blendlichtquelle 24 ist horizontal-seitlich und oder vertikal-seitlich in einem Abstand von der Blickfixierungsmarkierung 16 angeordnet. Der seitliche Abstand ist so bemessen, dass mit der Blendlichtquelle 24 ein Lichtbündel erzeugbar ist, das die Testperson blendet, wenn deren Blickrichtung 26 von dem vorbestimmten Ort 20 der Testperson ausgehend zur Blickfixierungsmarkierung 16 verläuft. Der Abstand ist bevorzugt so groß, dass die Blendlichtquelle 24 von dem genannten Ort 20 aus bei auf die Blickfixierungsmarkierung 16 gerichtetem Blick im Bereich des Peripheren Sehens der Testperson liegt.

Die Lichtstärke und Leuchtdichte der Blendlichtquelle 24 ist größer als die Helligkeit des dargestellten Sehzeichens 14. Die Helligkeit der Blendlichtquelle 24 ist insbesondere so groß, dass ein Sehzeichen 14, das auf der Präsentationsfläche 12 örtlich so dargestellt wird, dass sich die Blendlichtquelle 24, vom Ort 20 aus betrachtet, vor dem Zentrum des dargestellten Sehzeichens 14 befindet, von der Blendlichtquelle 24 überstrahlt wird und damit nicht erkennbar ist. Das Sehzeichen 14 nimmt dabei auf der Präsentationsfläche 12 eine Fläche ein, die rein geometrisch nur zu einem Teil von der Blendlichtquelle 24 verdeckt wird, so dass das Sehzeichen 14 bei ausgeschalteter oder in ihrer Helligkeit reduzierter Blendlichtquelle 24 durchaus wahrnehmbar wäre.

Die Blendwirkung nimmt mit zunehmendem seitlichen Abstand eingeblendeter Sehzeichen 14 von der Blendlichtquelle 24 ab, so dass Sehzeichen 14, deren seitlicher Abstand von der Blendlichtquelle 24 einen zu ermittelnden, Testpersonindividuellen Schwellenabstand überschreitet, für die Testperson in deren peripheren Sehfeld wahrnehmbar werden.

Die Erfindung erlaubt eine ortsaufgelöste Bestimmung solcher Schwellenabstände z.B. dadurch, dass in einem Raster von {bevorzugt konzentrisch) um die Blendlichtquelle 24 angeordneten Positionen mindestens ein Sehzeichen 14 eingeblendet wird, das definierte Eigenschaften wie Sehwinkel {d.h. Größe) und Kontrast besitzt. Diese Schwellenabstände sind Beispiele von zu akquirierenden Daten.

Eine Blickrichtungserkennungsvorrichtung 28 (eye tracking System) umfasst zwei Kameras 30, 32, die beide auf den genannten Ort 20 ausgerichtet sind und ihre Bildsignale an das Steuergerät 22 übermitteln. Das Steuergerät 22 ist dazu eingerichtet, durch eine Bildauswertung die Blickrichtung 26 und insbesondere Änderungen der Blickrichtung 26 einer Testperson, die sich am genannten Ort 20 befindet, zu ermitteln.

Die Blickrichtungserkennungsvorrichtung 28 umfasst damit die beiden Kameras 30, 32 und das Steuergerät 22. Eye tracking Systeme und Details der Bildauswertung werden hier als bekannt vorausgesetzt. Eine für das Beibehalten der Blickrichtung 26 der Testperson hilfreiche Positionierung des Kopfes der Testperson kann ggf. durch eine Kinn-/Stirnstütze zusätzlich verbessert werden.

Die erfindungsgemäße Testvorrichtung 10 weist darüber hinaus eine Eingabevorrichtung 34 auf, die dazu eingerichtet ist, Reaktionen der Testperson zu erfassen und zur Aufzeichnung an das Steuergerät 22 zu übergeben. Das eine Reaktion auslösende Ereignis ist zum Beispiel die Wahrnehmung eines Sehzeichens 14 auf der Präsentationsfläche 12 durch die Testperson. Die Reaktion besteht zum Beispiel darin, dass die Testperson den Ort des Sehzeichens 14 auf der Präsentationsfläche 12 angibt. In einem Beispiel kann die Eingabevorrichtung 34 eine Matrix von Tasten 36 aufweisen, von denen jede einen bestimmten Bereich auf der Präsentationsfläche 12 repräsentiert. Die Testperson gibt den Bereich, in dem sie das Sehzeichen 14 wahrgenommen hat, durch Betätigen der diesem Bereich zugeordneten Taste 36 an.

In einer anderen Ausgestaltung wird die Testperson aufgefordert, Ort und/oder Orientierung wahrgenommener Sehzeichen 14 in vorgegebenen Stichworten zu beschreiben, die von Messpersonal der Testvorrichtung 10 oder von einer Spracherkennung, die z.B. ebenfalls durch das Steuergerät 22 erfolgt, aufgenommen werden.

Dadurch wird im Dialog mit der Testperson für jede Position eines auf der Präsentationsfläche 12 dargestellten Sehzeichens 14 festgestellt, ob die Testperson das eingeblendete Sehzeichen 14 wahrnimmt und ggf. seine Orientierung im Raum (z.B. Die Lage einer Lücke eines Landolt-Ringes) richtig erkennt.

In Bezug auf die Verfahrensaspekte erfolgt bei eingeschalteter Blendlichtquelle 24 sequenziell ein Darstellen von wenigstens einem Sehzeichen 14 an mehreren um die Blendlichtquelle 24 herum verteilten und in dem Sehbereich der Testperson liegenden Positionen, wobei die Darstellung jeweils für eine vorbestimmte Präsentationsdauer erfolgt. Eine Darstellungssequenz beginnt jeweils mit der Darstellung eines Sehzeichens 14 am Ort der Blendlichtquelle 24. Dort wird das Sehzeichen 14 von der Blendlichtquelle 24 überstrahlt und ist daher für die Testperson nicht wahrnehmbar. Anschließend wird der laterale Abstand des dargestellten Sehzeichens 14 zur Blendlichtquelle 24 sukzessive in diskreten Schritten oder bevorzugt kontinuierlich vergrößert, so dass sich das Sehzeichen 14 allmählich aus dem Blendbereich herausbewegt und, je nach Testperson, früher oder später (d. h. bei kleineren oder größeren Schwellenabständen zur Blendlichtquelle 24) von der Testperson wahrgenommen werden kann. Alternativ oder ergänzend kann der Sehwinkel, unter dem das Sehzeichen für die Testperson sichtbar ist, also dessen Größe, und/oder dessen Kontrast variiert werden.

Bei der statischen Darbietung kann die Präsentationszeit, bei der dynamischen Darstellung auch dessen Winkelgeschwindigkeit variiert werden. Mit dem Begriff der Winkelgeschwindigkeit ist hier die "Bahngeschwindigkeit" gemeint, mit der sich der Stimulus auf der Projektionsfläche bewegt. Da diese Bewegung auf die Untersuchungsentfernung, z.B. den Kuppelradius einer Projektionsfläche, bezogen wird, erscheint hier der Begriff "Winkelgeschwindigkeit" am angemessensten.

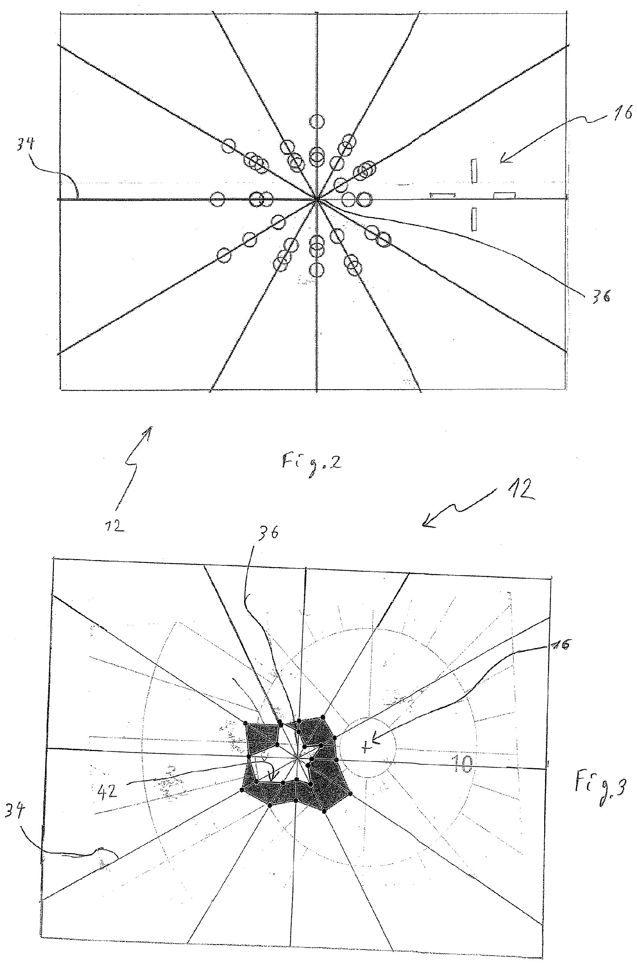

Figur 2 zeigt eine Verteilung von mit der Erfindung akquirierbaren Daten auf einer Präsentationsfläche 12. Zwölf Bahnkurven 38 schneiden sich in einem allen Bahnkurven 38 gemeinsamen Schnittpunkt 40 (Ursprung). Der Schnittpunkt 40 markiert den Ort der Blendlichtquelle 24. Auf der horizontalen Bahnkurve 38 ist ferner eine Blickfixierungsmarkierung 16 dargestellt. Jeweils zwei einander unmittelbar benachbarte Bahnkurven 38 schließen einen Winkel ein, der für alle Paare unmittelbar benachbarter Bahnkurven 38 gleich ist. Der Winkel beträgt 360° geteilt durch die Zahl der Bahnkurven 38, so dass der Winkel im dargestellten Beispiel 30° beträgt.

Bei einem bevorzugten Ausführungsbeispiel des Verfahrens wird jeweils ein Sehzeichen 14 zunächst in einer initialen Position, bevorzugt am Schnittpunkt/Ursprung 40 der Bahnkurven 38 auf der Präsentationsfläche 12 dargestellt. In der initialen Position wird das Sehzeichen 14 dabei durch die eingeschaltete Blendlichtquelle 24 überstrahlt. Die flächenmäßige Ausdehnung des Sehzeichens 14 ist bevorzugt größer als die Blendlichtquelle 24, so dass das Sehzeichen 14 prinzipiell sichtbar ist. Die Darstellung des Sehzeichens erfolgt allerdings mit einer Leuchtdichte, die wesentlich kleiner als die Leuchtdichte der Blendlichtquelle 24 ist. Als Folge überstrahlt die Blendlichtquelle 24 das Sehzeichen 14 in dieser Position, so dass das Sehzeichen 14 für die Testperson, deren Blickrichtung 26 auf die Blickfixierungsmarkierung 16 fixiert ist, nicht wahrnehmbar ist.

Ausgehend von dieser initialen Position wird das Sehzeichen 14 in diskreten Schritten oder kontinuierlich längs einer Bahnkurve 38 aus der initialen Position heraus bewegt. Durch die damit einhergehende Zunahme des Abstands des Sehzeichens 14 von der Blendlichtquelle 24 rückt das Sehzeichen 14 allmählich in einen Bereich hinein, in dem es von der Blendlichtquelle 24 nicht mehr überstrahlt und als Folge von der Testperson durch Peripheres Sehen wahrgenommen werden kann. Die Testperson reagiert auf eine solche Wahrnehmung mit der Betätigung der Eingabevorrichtung 34 (oder einer anderen mit dem Messpersonal verabredeten Reaktion).

Dem Steuergerät 22, das die Darstellung der Sehzeichen 14 steuert, sind die jeweiligen Positionsdaten des Sehzeichens 14 bekannt. Das Steuergerät 22 speichert die aktuellen Positionsdaten jeweils dann ab, wenn die Eingabevorrichtung 34 betätigt wird.

Dieser Ablauf wird für jede Bahnkurve 38 ggf. mehrfach wiederholt. Es ergibt sich dadurch zum Beispiel eine Datenverteilung, wie sie in der Figur 2 als Kreiswolke dargestellt ist. Der Mittelpunkt von jedem der Kreise gibt den Ort auf der Präsentationsfläche 12 an, für den die Testperson eine Reaktion auf eine Wahrnehmung eines Sehzeichens 14 an diesem Ort eingegeben hat. Dieser Ort wird z.B. durch Angabe der Daten charakterisiert, welche die jeweilige Bahnkurve 34 und den jeweiligen Abstand kennzeichnen. Ein solches Wertepaar stellt ein Beispiel erfindungsgemäß akquirierter Daten dar. Eine Bewertung von Größe und Lage des von Kreisen freien Bereichs im Inneren der Kreiswolke erlaubt Rückschlüsse auf eine Testpersonindividuelle Beeinträchtigung ihres Peripheren Sehens durch die Blendlichtquelle unter standardisierten und reproduzierbaren Testbedingungen.

Um die Wahrscheinlichkeit fehlerhafter Erkennungen zu verringern, kann die Testperson dazu aufgefordert werden, nicht nur anzugeben, dass sie ein Sehzeichen 14 wahrnimmt, sondern zusätzlich eine Eigenschaft des wahrgenommenen Sehzeichens 14 anzugeben. Eine solche Eigenschaft kann zum Beispiel bei Landolt-Ringen als Sehzeichen 14 die Lage der Lücke des Landolt-Rings sein, die sich zum Beispiel oben, rechts oben, rechts, rechts unten, unten usw. befinden kann.

Stimmt die Eingabe nicht mit der vom Steuergerät vorgegebenen wahren Lage überein, wird die Eingabe vom Steuergerät verworfen.

Nach der für mindestens ein Sehzeichen 14 durchgeführten Akquisition von Daten wird die Akquisition zum Beispiel dadurch fortgesetzt, dass mindestens eine Blendlichtquelle 24 entweder auf eine andere Position bewegt wird, oder es wird von einer Vielzahl auf verschiedenen Positionen vorhandenen Blendlichtquellen 24 mindestens eine ausgeschaltet und/oder mindestens eine eingeschaltet. Die Messung kann anschließend fortgesetzt werden, indem ein anderes Sehzeichen 14 eingeblendet wird und die Testperson nun Auskunft darüber gibt, ob und wie sie das Sehzeichen 14 erkennen kann.

Das Darstellen der Sehzeichen 14 erfolgt bei eingeschalteter Blendlichtquelle 24 und für eine vorbestimmte Präsentationsdauer. Wenn innerhalb der Präsentationdauer keine Reaktion der Testperson erfolgt, werden für die betreffende Präsentationsdauer auch keine Daten akquiriert.

Die Messung endet, nachdem die Daten für eine bestimmte Anzahl an Positionen/Muster von Blendlichtquellen 24 und Sehzeichen 14 akquiriert worden sind oder das Messpersonal die Messung von Hand abbricht.

Aus den längs jeweils einer individuellen Bahnkurve 34 akquirierten Abstandsdaten wird bevorzugt durch statistische Bewertung wie Mittelwertbildung oder Medianbildung für diese Bahnkurve 34 ein Punkt bestimmt, dessen Abstand vom Schnittpunkt 40 eine statistisch abgesicherte Entfernung von der Blendlichtquelle 24 kennzeichnet, bis zu der die Blendlichtquelle 24 für die betreffende Testperson eine Erkennung des Sehzeichens 14 verhindert hat.

Wenn das Sehzeichen 14 bei der Bestimmung des Abstandes vom Schnittpunkt 40 mit vorgegebener Winkelgeschwindigkeit kontinuierlich entlang einer Bahnkurve 34 bewegt worden ist, liegt zwischen dem Moment der Wahrnehmung und der Betätigung der Eingabevorrichtung 34 (oder einer anderen Reaktion) eine Reaktionszeit der Testperson. Wenn diese Reaktionszeit bei der Bestimmung des Abstandes auf der Bahnkurve 34, bei dem das Sehzeichen 14 wahrgenommen wurde, nicht berücksichtigt wird, ergibt sich ein systematischer Fehler in Form eines zu großen Abstands. Dies würde einer stärkeren Beeinträchtigung des Peripheren Sehens durch die Blendlichtquelle entsprechen als tatsächlich vorliegt. Zur Abhilfe wird die Reaktionszeit der Testperson bestimmt und bei der Bestimmung des genannten Punktes der Bahnkurve berücksichtigt. Die Berücksichtigung erfolgt dadurch, dass der Teil der Bahnkurve, den das Sehzeichen in der Reaktionszeit zurückgelegt als Produkt aus Reaktionszeit und Sehzeichenwinkelgeschwindigkeit bestimmt wird und dass der zunächst ohne Berücksichtigung der Reaktionszeit bestimmte Punkt der Bahnkurve um diesen Teil der Bahnkurve in Richtung zu der initialen Position verlegt wird.

Figur 3 zeigt die Präsentationsfläche aus der Figur 2 mit Daten, die unter zwei verschiedenen Randbedingungen akquiriert worden sind. Eine erste Punktmenge ist zu einer ersten, inneren geschlossenen Isoptere verbunden. Die diesbezüglichen Daten (Lage des Sehzeichens auf der betreffenden Bahnkurve bei korrekter Wahrnehmung und Reaktion der Testperson) sind ohne eine Sehhilfe bestimmt worden. Die innerhalb dieser ersten Schleife 42 liegende Fläche stellt ein mögliches Maß dar, das die Blendungsempfindlichkeit des Sehsinns einer Testperson im Bereich des Peripheren Sehens kennzeichnet.

Eine zweite Punktmenge ist zu einer zweiten, äußeren geschlossenen Isoptere 44 verbunden. Die diesbezüglichen Daten (Lage des Sehzeichens auf der betreffenden Bahnkurve bei korrekter Wahrnehmung und Reaktion der Testperson) sind mit einer das Sehvermögen zu Veranschaulichungszwecken bewusst verschlechternden Brille bestimmt worden. Die innerhalb dieser zweiten Isoptere 44 liegende Fläche stellt ein weiteres mögliches Maß dar, das die Empfindlichkeit des Sehsinns einer Testperson auf eine Blendung kennzeichnet.

Bei mehreren Isopteren ist prinzipiell auch eine volumetrische Quantifizierung ("Defektvolumen" möglich): Zum Beispiel stellt die zwischen den beiden Isopteren 44, 42 liegende Differenzfläche ein Maß für die durch die (hier bewusst verschlechternde) Brille verursachte Änderung der Fähigkeit der Testperson zum Peripheren Sehen dar.

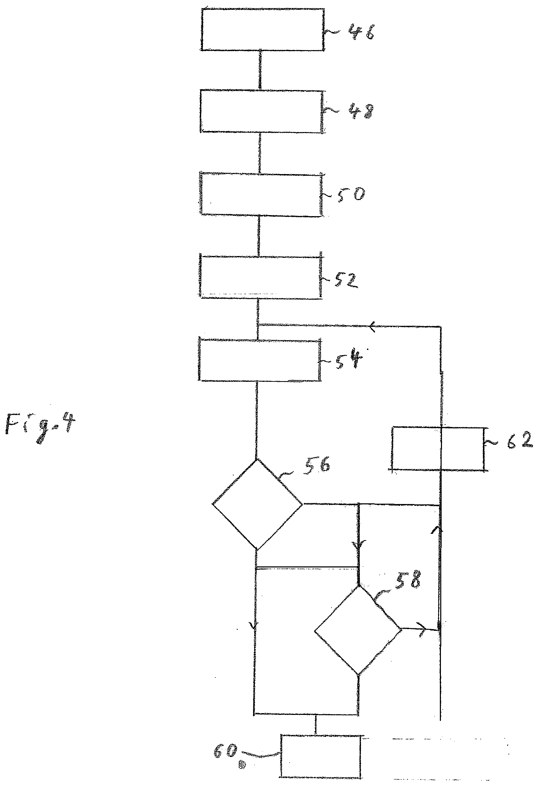

Figur 4 zeigt ein Flussdiagramm als Ausführungsbeispiel eines erfindungsgemäßen Verfahrens. Mit dem im Folgenden verwendeten Begriff des Schrittes kann auch eine Schrittfolge, also ein Unterprogramm gemeint sein. Die Reihenfolge der Schritte/Schrittfolgen muss nicht der bei dem folgenden Ausführungsbeispiel gewählten Reihenfolge entsprechen.

In einem ersten Schritt 46 wird eine Testvorrichtung 10 bereitgestellt, die die Präsentationsfläche 12, wenigstens eine Blendlichtquelle 24, eine Eingabevorrichtung 34, eine Blickrichtungserkennungsvorrichtung 28 und ein Steuergerät 22 aufweist.

In einem zweiten Schritt 48 wird die Testperson, deren Daten ermittelt werden sollen, so platziert, dass die Blickfixierungsmarkierung 16 in einem zentralen Bereich des Sichtfeldes der Testperson liegt. Dabei blickt die Testperson bevorzugt geradeaus.

In einem dritten Schritt 50 wird die Testperson angewiesen, ihren Blick auf die Blickfixierungsmarkierung 16 zu richten und nicht von der Blickfixierungsmarkierung 16 zu lösen. Darüber hinaus wird sie dazu angewiesen, eine bestimmte Reaktion zu zeigen, wenn sie ein Sehzeichen 14 auf der Präsentationsfläche 12 wahrnimmt.

In einem vierten Schritt 52 wird die Blendlichtquelle 24 eingeschaltet. Dieser Schritt kann auch zu einem anderen Zeitpunkt erfolgen, bspw. nach dem ersten Schritt 46 oder als Bestandteil des ersten Schrittes 46.

In einem fünften Schritt 54 erfolgt bei eingeschalteter Blendlichtquelle 24 eine Darstellung von Sehzeichen 14 in verschiedenen Positionen einer vorbestimmten Bahnkurve 34 auf der Präsentationsfläche 12. Die Präsentationsdauer des mindestens einen Sehzeichens 14 ist definiert und voreinstellbar und kann variiert werden.

In einem sechsten Schritt 56 erfolgt ein fortwährendes Überwachen der Blickrichtung 26 der Testperson mit der Blickrichtungserkennungsvorrichtung 28.

In einem siebten Schritt 58 erfolgt fortwährend ein Überwachen der Testperson auf das Zeigen der bestimmten Reaktion.

In einem achten Schritt 60 erfolgt ein Beenden der Darstellung des Sehzeichens dann, wenn mit der Blickrichtungserkennungsvorrichtung erkannt wird, dass sich der Blick der Testperson zwischen der Darstellung des Sehzeichens und der davon ausgelösten Reaktion der Testperson von der Blickfixierungsmarkierung gelöst hat. In diesem Fall werden keine Daten akquiriert.

Eine Akquisition von Daten, d.h. ein Erfassen und Speichern von Daten erfolgt in einem neunten Schritt 62 dann, wenn sich der Blick der Testperson zwischen der Darstellung des Sehzeichens und der davon ausgelösten Reaktion der Testperson nicht von der Blickfixierungsmarkierung gelöst hat. Bei den Daten handelt es sich bevorzugt um Daten, welche die Position des Sehzeichens in dem Moment kennzeichnen, in dem die Testperson die Reaktion zeigt.

Die Darstellung der Sehzeichen erfolgt an mehreren um die Blendlichtquelle herum verteilten und in dem Sehbereich liegenden Positionen, die insofern Prüforte bilden. Die Prüforte sind konzentrisch um die Blendlichtquelle herum angeordnet.

In einer Ausgestaltung wird für jeden Prüfort der Sehwinkel, unter dem das Sehzeichen für die Testperson sichtbar ist, oder der Sehzeichenkontrast für eine vorgegebene Sehschärfestufe variiert.

Nach der Messung von mindestens einem Sehzeichen, wird die Messung fortgesetzt, indem mindestens eine Blendlichtquelle entweder auf eine andere Position bewegt wird, oder es wird von einer Vielzahl auf verschiedenen Positionen vorhandenen Blendlichtquellen mindestens eine ausgeschaltet und/oder mindestens eine eingeschaltet.

Die Messung endet, nachdem eine bestimmte Anzahl an Positionen/Muster von Blendlichtquellen und Sehzeichen gemessen wurde oder das Messpersonal die Messung von Hand abbricht.

## Patentansprüche

1. Verfahren zur Akquisition von Daten, die sich auf eine Blendungsempfindlichkeit einer Testperson beziehen, **dadurch gekennzeichnet, dass** das Verfahren die folgenden Schritte aufweist:
a) Bereitstellen einer Testvorrichtung (10), die wenigstens die folgenden Elemente aufweist:
a1) eine Präsentationsfläche (12), auf der die Sehzeichen (14) in variablen Positionen und wenigstens eine ortsfeste Blickfixierungsmarkierung (16) darstellbar sind;
a2) wenigstens eine Blendlichtquelle (24), die seitlich versetzt zu einer Blickrichtung (26) der Testperson so angeordnet ist, dass mit ihr ein Lichtbündel erzeugbar ist, das die Testperson blendet, wenn die Blickrichtung (26) zwischen einem vorbestimmten Ort (20) der Testperson und der Blickfixierungsmarkierung (16) verläuft;
a3) eine Eingabevorrichtung (34) mit der Reaktionen der Testperson erfassbar und aufzeichnungsbar sind; und
a4) eine Blickrichtungserkennungsvorrichtung (28), mit der eine Blickrichtung (26) der Testperson detektierbar ist;
wobei das Verfahren die folgenden Schritte umfasst:
b) Platzieren der Testperson so, dass die Blickfixierungsmarkierung (16) in einem zentralen Bereich des Sichtfeldes der Testperson liegt;
c) Anweisen der Testperson, ihren Blick auf die Blickfixierungsmarkierung (16) zu richten und nicht von der Blickfixierungsmarkierung (16) zu lösen;
d) Anweisen der Testperson, eine bestimmte Reaktion zu zeigen, wenn sie ein Sehzeichen (14) auf der Präsentationsfläche (12) wahrnimmt;
e) Einschalten der Blendlichtquelle (24);
f) bei eingeschalteter Blendlichtquelle (24), Darstellen eines Sehzeichens (14) in verschiedenen Positionen einer vorbestimmten Bahnkurve (34) auf der Präsentationsfläche (12),
g) fortwährendes Überwachen der Blickrichtung (26) der Testperson mit der Blickrichtungserkennungsvorrichtung (28) ;
h) Überwachen der Testperson auf das Zeigen der bestimmten Reaktion;
i) Beenden der Darstellung des Sehzeichens (14), wenn mit der Blickrichtungserkennungsvorrichtung (28) erkannt wird, dass sich der Blick der Testperson zwischen der Darstellung des Sehzeichens (14) und der davon ausgelösten Reaktion der Testperson von der Blickfixierungsmarkierung (16) gelöst hat; und
j) Erfassen und Speichern von Daten, welche die Position des Sehzeichens (14) in dem Moment kennzeichnen, in dem die Testperson die Reaktion zeigt, nur dann, wenn sich der Blick der Testperson zwischen der Darstellung des Sehzeichens (14) und der davon ausgelösten Reaktion der Testperson nicht von der Blickfixierungsmarkierung (16) gelöst hat.

2. Verfahren nach Anspruch 1, **dadurch gekennzeichnet, dass** die Blendlichtquelle (24) bezüglich einer auf die Blickfixierungsmarkierung (16) gerichteten Blickrichtung (26) der Testperson seitlich versetzt in einem Bereich des Peripheren Sehens positioniert wird.

3. Verfahren nach Anspruch 2, **dadurch gekennzeichnet, dass** die Blendlichtquelle (24) so positioniert wird, dass sie eine zentrale/foveale Blendung erzeugt.

4. Verfahren nach einem der vorhergehenden Ansprüche, **dadurch gekennzeichnet, dass** das Sehzeichen (14) einen 8-Positionen Landolt-Ring und/oder ein davon verschiedenes grafisches Symbol und/oder wenigstens eine Zahl und/oder wenigstens einen Buchstaben aufweist.

5. Verfahren nach einem der vorhergehenden Ansprüche, **dadurch gekennzeichnet, dass** das Sehzeichen (14) mit vorgegebener Geschwindigkeit kontinuierlich entlang einer Bahnkurve (34) bewegt wird, deren Ursprung von der Testperson aus gesehen von der Blendlichtquelle (24) verdeckt wird.

6. Verfahren nach einem der vorhergehenden Ansprüche, **dadurch gekennzeichnet, dass** aus den längs jeweils einer Bahnkurve (34)erfassten Daten durch statistische Bewertung wie Mittelwertbildung oder Medianbildung von Schwellenwertabständen für diese Bahnkurve (34) ein Punkt bestimmt wird, dessen Abstand vom Ursprung für diese Bahnkurve (34) eine Entfernung kennzeichnet, bis zu der die Blendlichtquelle (24) für die betreffende Testperson eine Erkennung des Sehzeichens (14) verhindert hat.

7. Verfahren nach Anspruch 6, **dadurch gekennzeichnet, dass** bei der Bestimmung der Daten eine Reaktionszeit der Testperson berücksichtigt wird.

8. Verfahren nach Anspruch 7, **dadurch gekennzeichnet, dass** die Berücksichtigung dadurch erfolgt, dass der Teil der Bahnkurve (34), den das Sehzeichen (14) in der Reaktionszeit zurückgelegt hat, als Produkt aus Reaktionszeit und Sehzeichengeschwindigkeit bestimmt wird und dass der zunächst ohne Berücksichtigung der Reaktionszeit bestimmte Punkt der Bahnkurve (34) um diesen Teil der Bahnkurve (34) in Richtung zu der initialen Position verlegt wird.

9. Verfahren nach einem der vorhergehenden Ansprüche, **dadurch gekennzeichnet, dass** die Schrittfolge des Anspruchs 1 beendet wird, wenn sich der Blick der Testperson von der Blickfixierungsmarke (16) löst und alle ggf. bereits für dieses Sehzeichen (14) festgestellte Wahrnehmungen verworfen werden.

10. Verfahren nach einem der vorhergehenden Ansprüche, **dadurch gekennzeichnet, dass** das Darstellen eines Sehzeichens (14) für eine vorbestimmte Präsentationsdauer erfolgt und dass dann, wenn innerhalb der Präsentationdauer keine Reaktion der Testperson erfolgt, für die betreffende Präsentationsdauer auch keine Daten akquiriert werden.

11. Verfahren nach einem der vorhergehenden Ansprüche, **dadurch gekennzeichnet, dass** die Position der Blendlichtquelle (24) nach einer bei einer ersten Blendlichtquellenposition erfolgten Akquisition von Daten verändert wird und das Verfahren für verschiedene Positionen der Blendlichtquelle (24) durchgeführt wird.

12. Verfahren nach einem der vorhergehenden Ansprüche, **dadurch gekennzeichnet, dass** die spektrophoto-radiometrischen Eigenschaften der Blendlichtquelle (24) variiert werden, und dass eine Akquisition von Daten für verschiedene spektrophoto-radiometrische Eigenschaften der Blendlichtquelle (24) erfolgt.

13. Testvorrichtung (10) zur Akquisition von Daten, die sich auf eine Blendungsempfindlichkeit einer Testperson beziehen, wobei die Testvorrichtung (10)
- wenigstens eine Präsentationsfläche (129 aufweist, in die Sehzeichen (14) in variablen Positionen darstellbar sind und in der wenigstens eine ortsfeste Blickfixierungsmarkierung (16) darstellbar ist,
- wenigstens eine Blendlichtquelle (24) aufweist, die seitlich versetzt zu einer Blickrichtung (26) der Testperson so angeordnet ist, dass mit ihr Lichtbündel erzeugbar sind, mit denen die Testperson geblendet wird, wenn die Blickrichtung (26) zwischen einem vorbestimmten Ort (20) der Testperson und der Blickfixierungsmarkierung (16) verläuft,
- eine Eingabevorrichtung (34) aufweist, mit dem Reaktionen der Testperson erfassbar und aufzeichnungsbar sind,
- eine Blickrichtungserkennungsvorrichtung (28) aufweist, mit der eine Blickrichtung (26) der Testperson detektierbar ist,
- dazu eingerichtet ist die Blickfixierungsmarkierung (16) in einem zentralen Bereich des Sichtfeldes der Testperson zu erzeugen,
- bei eingeschalteter Blendlichtquelle (24), die Sehzeichen (14) in verschiedenen Positionen auf der Präsentationsfläche (12) darzustellen,
- Reaktionen der Testperson auf die Darstellung der Sehzeichen (14) zu erfassen und die beim Erfassen der Reaktion aktuelle Position des Sehzeichens (14) zu speichern,
- die Blickrichtung (16) der Testperson mit der Blickrichtungserkennungsvorrichtung (28) fortwährend zu Überwachen und die Darstellung eines Sehzeichens zu beenden, wenn mit der Blickrichtungserkennungsvorrichtung (28) erkannt wird, dass sich der Blick der Testperson zwischen der Darstellung des Sehzeichens (14) und der davon ausgelösten Reaktion der Testperson von der Blickfixierungsmarkierung (16) gelöst hat
- und die bei einer Reaktion der Testperson auf die Darstellung eines Sehzeichens (14) vorgesehene Speicherung der Position nur dann durchzuführen, wenn sich der Blick der Testperson zwischen der Darstellung des Sehzeichens (14) und der davon ausgelösten Reaktion der Testperson nicht von der Blickfixierungsmarkierung (16) gelöst hat.

14. Testvorrichtung (10) nach Anspruch 13, **dadurch gekennzeichnet, dass** sie dazu eingerichtet ist, ein Verfahren nach einem der Ansprüche 2 bis 13 durchzuführen.

## Claims

1. Method for acquiring data relating to glare sensitivity of a test person, **characterized in that** the method comprises the following steps:
a) providing a test device (10) having at least the following elements:
a1) a presentation surface (12) on which the optotypes (14) in variable positions and at least one stationary gaze fixation marking (16) can be displayed;
a2) at least one glare source (24) arranged laterally offset to a gaze direction (26) of the test person, such that it can be used to generate a light beam that dazzles the test person when the gaze direction (26) extends between a predetermined location (20) of the test person and the gaze fixation marking (16);
a3) an input device (34), by means of which responses of the test person can be detected and recorded; and
a4) a gaze direction detection device (28), by means of which a gaze direction (26) of the test person can be detected;
the method comprising the following steps:
b) placing the test person such that the gaze fixation marking (16) is in a central region of the field of view of the test person;
c) instructing the test person to direct their gaze to the gaze fixation marking (16) and not to remove it from the gaze fixation marking (16);
d) instructing the test person to show a particular response when they perceive a optotype (14) on the presentation surface (12);
e) switching on the glare source (24);
f) when the glare source (24) is switched on, displaying a optotype (14) on the presentation surface (12) in different positions of a predetermined trajectory (34),
g) continuously monitoring the gaze direction (26) of the test person with the gaze direction detection device (28);
h) monitoring the test person to indicate the particular response;
i) terminating the display of the optotype (14) when it is detected by means of the gaze direction detection device (28) that the gaze of the test person has been removed from the gaze fixation marking (16) between the display of the optotype (14) and the response of the test person triggered thereby; and
j) acquiring and storing data which mark the position of the optotype (14) at the moment in which the test person shows the reaction only when the gaze of the test person has not been removed from the gaze fixation marking (16) between the display of the optotype (14) and the response of the test person triggered thereby.

2. Method according to claim 1, **characterized in that** the glare source (24) is positioned, in a region of peripheral sight, laterally offset with respect to a gaze direction (26) of the test person directed to the gaze fixation marking (16).

3. Method according to claim 2, **characterized in that** the glare source (24) is positioned such that it generates a central/foveal glare.

4. Method according to any of the preceding claims, **characterized in that** the optotype (14) has an 8-position Landolt ring and/or a graphical symbol different therefrom and/or at least one number and/or at least one letter.

5. Method according to any of the preceding claims, **characterized in that** the optotype (14) is moved continuously along a trajectory (34) at a predetermined speed, the origin of said trajectory, when viewed by the test person, being concealed by the glare source (24).

6. Method according to any of the preceding claims, **characterized in that** from the data acquired in each case along a trajectory (34) and by statistical evaluation such as averaging or median formation of threshold distances for this trajectory (34), a point is determined of which the distance from the origin for this trajectory (34) designates a distance up to which the glare source (24) has prevented recognition of the optotype (14) for the test person concerned.

7. Method according to claim 6, **characterized in that** a response time of the test person is taken into account when determining the data.

8. Method according to claim 7, **characterized in that** the taking into account takes place by the part of the trajectory (34) which the optotype (14) has covered in the reaction time being determined as a product of response time and optotype velocity, and by the point of the trajectory (34) initially determined without taking into account the response time being displaced around this part of the trajectory (34) in the direction of the initial position.

9. Method according to any of the preceding claims, **characterized in that** the step sequence of claim 1 is terminated when the gaze of the test person is removed from the gaze fixation marking (16) and all of the perceptions that may have already been detected for this optotype (14) are rejected.

10. Method according to any of the preceding claims, **characterized in that** a optotype (14) is displayed for a predetermined presentation period and **in that** if there is no reaction of the test person within the presentation period, no data is acquired for the relevant presentation period.

11. Method according to any of the preceding claims, **characterized in that** the position of the glare source (24) is changed after acquisition of data at a first glare source position, and the method is carried out for different positions of the glare source (24).

12. Method according to any of the preceding claims, **characterized in that** the spectroradiometric properties of the glare source (24) are varied, and **in that** data is acquired for various spectroradiometric properties of the glare source (24).

13. Test device (10) for acquiring data relating to glare sensitivity of a test person, wherein the test device (10) has at least one presentation surface (12) in which optotypes (14) can be displayed in variable positions and in which at least one stationary gaze fixation marking (16) can be displayed,
- has at least one glare source (24) arranged laterally offset to a gaze direction (26) of the test person, such that it can be used to generate a light beam that dazzles the test person when the gaze direction (26) extends between a predetermined location (20) of the test person and the gaze fixation marking (16),
- has an input device (34), by means of which responses of the test person can be detected and recorded,
- has a gaze direction detection device (28), by means of which a gaze direction (26) of the test person can be detected,
- is configured to generate the gaze fixation marking (16) in a central region of the field of view of the test person,
- when the glare source (24) is switched on, to display the optotype (14) on the presentation surface (12) in different positions,
- to detect responses of the test person to the display of the optotypes (14) and to store the current position of the optotype (14) when the reaction is detected,
- to continue to monitor the gaze direction (16) of the test person using the gaze direction detection device (28) and to terminate the display of a optotype when it is detected by means of the gaze direction detection device (28) that the gaze of the test person has been removed from the gaze fixation marking (16) between the display of the optotype (14) and the response of the test person triggered thereby
- and to carry out the storage of the position provided upon a response of the test person to the display of a optotype (14) only if the gaze of the test person has not been removed from the gaze fixation marking (16) between the display of the optotype (14) and the response of the test person triggered thereby.

14. Test device (10) according to claim 13, **characterized in that** it is configured to carry out a method according to any of claims 2 to 13.

## Revendications

1. Procédé d'acquisition de données, qui concernent une sensibilité à l'éblouissement d'une personne soumise à un test, **caractérisé en ce que** le procédé présente les étapes suivantes :
a) la fourniture d'un dispositif de test (10), qui présente au moins les éléments suivants :
a1) une surface de présentation (12), sur laquelle peuvent être exposés les optotypes (14) dans des positions variables et au moins un marquage de fixation de regard (16) fixe ;
a2) au moins une source de lumière aveuglante (24), qui est disposée de manière décalée latéralement par rapport à une direction de regard (26) de la personne soumise à un test, de sorte qu'un faisceau lumineux peut être produit avec celle-ci, qui éblouit la personne soumise à un test lorsque la direction de regard (26) s'étend entre un lieu (20) prédéfini de la personne soumise à un test et le marquage de fixation de regard (16) ;
a3) un dispositif d'entrée (34) avec lequel les réactions de la personne soumise à un test peuvent être acquises et peuvent être enregistrées ; et
a4) un dispositif de reconnaissance de regard (28), avec lequel une direction de regard (26) de la personne soumise à un test peut être détectée ;
dans lequel le procédé comprend les étapes suivantes :
b) le placement de la personne soumise à un test, de sorte que le marquage de fixation de regard (16) se situe dans une zone centrale du champ de vision de la personne soumise à un test ;
c) le fait de donner l'instruction à la personne soumise à un test de diriger son regard sur le marquage de fixation de regard (16) et de ne pas le détacher du marquage de fixation de regard (16) ;
d) le fait de donner l'instruction à la personne soumise à un test de manifester une réaction définie lorsqu'elle perçoit un optotype (14) sur la surface de présentation (12) ;
e) la mise en circuit de la source de lumière aveuglante (24) ;
f) lorsque la source de lumière aveuglante (24) est mise en circuit, l'exposition d'un optotype (14) dans différentes positions d'une trajectoire (34) prédéfinie sur la surface de présentation (12),
g) la surveillance continuelle de la direction de regard (26) de la personne soumise à un test avec le dispositif de reconnaissance de direction de regard (28) ;
h) la surveillance de la personne soumise à un test concernant la manifestation de la réaction définie ;
i) l'achèvement de l'exposition de l'optotype (14), lorsqu'il est reconnu avec le dispositif de reconnaissance de direction de regard (28) que le regard de la personne soumise à un test s'est détaché du marquage de fixation de regard (16) entre l'exposition de l'optotype (14) et la réaction de la personne soumise à un test déclenchée par cela ; et
j) l'acquisition et la mise en mémoire de données, lesquelles caractérisent la position de l'optotype (14) au moment où la personne soumise à un test ne manifeste la réaction que lorsque le regard de la personne soumise à un test ne s'est pas détaché du marquage de fixation de regard (16) entre l'exposition de l'optotype (14) et la réaction de la personne soumise à un test déclenchée par cela.

2. Procédé selon la revendication 1, **caractérisé en ce que** la source de lumière aveuglante (24) est positionnée de manière décalée latéralement par rapport à une direction de regard (26) de la personne soumise à un test dirigée sur le marquage de fixation de regard (16) dans une zone de la vision périphérique.

3. Procédé selon la revendication 2, **caractérisé en ce que** la source de lumière aveuglante (24) est positionnée de sorte qu'elle produit un aveuglement central/fovéal.

4. Procédé selon l'une quelconque des revendications précédentes, **caractérisé en ce que** l'optotype (14) présente un anneau de Landolt à 8 positions et/ou un symbole graphique différent de celui-ci et/ou au moins un chiffre et/ou au moins une lettre.

5. Procédé selon l'une quelconque des revendications précédentes, **caractérisé en ce que** l'optotype (14) est déplacé à une vitesse prédéfinie de manière continue le long d'une trajectoire (34), dont l'origine vue à partir de la personne soumise à un test est cachée par la source de lumière aveuglante (24).

6. Procédé selon l'une quelconque des revendications précédentes, **caractérisé en ce qu'**à partir des données acquises le long de respectivement une trajectoire (34) par une évaluation statistique telle que la formation de moyenne ou formation de médiane d'écarts de valeur de seuil pour cette trajectoire (34), un point est déterminé, dont l'écart par rapport à l'origine pour cette trajectoire (34) caractérise un éloignement jusqu'auquel la source de lumière aveuglante (24) pour la personne soumise à un test concernée a empêché une reconnaissance de l'optotype (14).

7. Procédé selon la revendication 6, **caractérisé en ce que** lors de la détermination des données un temps de réaction de la personne soumise à un test est pris en compte.

8. Procédé selon la revendication 7, **caractérisé en ce que** la prise en compte s'effectue de sorte que la partie de la trajectoire (34) que l'optotype (14) a parcourue pendant le temps de réaction est déterminée comme produit du temps de réaction et de la vitesse d'optotype et que le point de la trajectoire (34) tout d'abord déterminé sans prise en compte du temps de réaction est déplacé de cette partie de la trajectoire (34) en direction de la position initiale.

9. Procédé selon l'une quelconque des revendications précédentes, **caractérisé en ce que** la séquence d'étapes de la revendication 1 est achevée lorsque le regard de la personne soumise à un test se détache du marquage de fixation de regard (16) et que toutes les perceptions éventuellement déjà identifiées pour cet optotype (14) ont été rejetées.

10. Procédé selon l'une quelconque des revendications précédentes, **caractérisé en ce que** la présentation d'un optotype (14) s'effectue pour une durée de présentation prédéfinie et que lorsque pendant la durée de présentation aucune réaction de la personne soumise à un test n'a lieu, aucune donnée n'est non plus acquise pour la durée de présentation concernée.

11. Procédé selon l'une quelconque des revendications précédentes, **caractérisé en ce que** la position de la source de lumière aveuglante (24) est modifiée après une acquisition de données effectuée pour une première position de source de lumière aveuglante et le procédé est mis en œuvre pour différentes positions de la source de lumière aveuglante (24).

12. Procédé selon l'une quelconque des revendications précédentes, **caractérisé en ce que** les propriétés spectrophoto-radiométriques de la source de lumière aveuglante (24) sont variées, et qu'une acquisition de données s'effectue pour différentes propriétés spectrophoto-radiométriques de la source de lumière aveuglante (24).

13. Dispositif de test (10) pour l'acquisition de données, qui concernent une sensibilité à l'éblouissement d'une personne soumise à un test, dans lequel le dispositif de test (10)
- présente au moins une surface de présentation (12), dans laquelle des optotypes (14) peuvent être exposés dans des positions variables et dans laquelle au moins un marquage de fixation de regard (16) fixe peut être exposé,
- au moins une source de lumière aveuglante (24), qui est disposée de manière décalée latéralement par rapport à une direction de regard (26) de la personne soumise à un test, de sorte que des faisceaux lumineux peuvent être produits avec celle-ci, avec lesquels la personne soumise à un test est aveuglée, lorsque la direction de regard (26) s'étend entre un emplacement (20) prédéfini de la personne soumise à un test et le marquage de fixation de regard (16),
- un dispositif d'entrée (34), avec lequel les réactions de la personne soumise à un test peuvent être acquises et peuvent être enregistrées,
- un dispositif de reconnaissance de regard (28), avec lequel une direction de regard (26) de la personne soumise à un test peut être détectée,
- est conçu pour produire le marquage de fixation de regard (16) dans une zone centrale du champ de vision de la personne soumise à un test,
- lorsque la source de lumière aveuglante (24) est mise en circuit, pour exposer les optotypes (14) dans différentes positions sur la surface de présentation (12),
- pour acquérir les réactions de la personne soumise à un test à l'exposition des optotypes (14) et pour mettre en mémoire la position actuelle de l'optotype (14) lors de l'acquisition de la réaction,
- pour surveiller de manière continuelle la direction de regard (28) de la personne soumise à un test avec le dispositif de reconnaissance de direction de regard (28) et pour achever l'exposition d'un optotype, lorsqu'il est reconnu avec le dispositif de reconnaissance de direction de regard (28) que le regard de la personne soumise à un test s'est détaché du marquage de fixation de regard (16) entre l'exposition de l'optotype (14) et la réaction de la personne soumise à un test déclenchée par celle-ci
- et pour ne mettre en œuvre la mise en mémoire de la position prévue lors d'une réaction de la personne soumise à un test à l'exposition d'un optotype (14) que lorsque le regard de la personne soumise à un test entre l'exposition de l'optotype (14) et la réaction de la personne soumise à un test déclenchée par celle-ci ne s'est pas détaché du marquage de fixation de regard (16).

14. Dispositif de test (10) selon la revendication 13, **caractérisé en ce qu'**il est conçu pour mettre en œuvre un procédé selon l'une quelconque des revendications 2 à 13.
